# EUROPEAN PATENT APPLICATION

(11) **EP 4 778 566 A1**
(43) Date of publication of application: **22.07.2026**
(21) Application number: 25152286.8
(22) Date of filing: 16.01.2025
(51) Int. Cl.: A61N 1/368

(54) **IMPLANTABLE MEDICAL DEVICE FOR CARDIAC RESYNCHRONIZATION THERAPY FEATURING AN AUTOMATIC ADAPTATION OF A STIMULATION PARAMETER**

(71) Applicant: BIOTRONIK SE & Co. KG, 12359 Berlin (DE)
(72) Inventor: Dörr, Thomas, 12437 Berlin (DE); Schneider, Peter, 12527 Berlin (DE); Becker, Frank, 10409 Berlin (DE); Bonim, Thomas, 13435 Berlin (DE)
(74) Representative: Biotronik Corporate Services SE

(57) **Abstract**

The invention relates to an implantable medical device for stimulating a human or animal heart that comprises a proximal electrode pole (213), a first distal electrode pole (203), and a second distal electrode pole (223). During operation, the implantable medical device performs the following steps: a) detecting (500), with the proximal electrode pole (213), an intrinsic atrial contraction of the heart (1) to be stimulated or stimulating (500), with the proximal electrode pole (213), the atrium (2) of the heart (1) to be stimulated; b) detecting (530), with the first distal electrode pole (203) and with the second distal electrode pole (223), an intrinsic ventricular contraction of the heart (1) to be stimulated; c) determining (530) a first intrinsic atrioventricular conduction time between i) the intrinsic atrial contraction or the stimulation of the atrium and ii) the intrinsic ventricular contraction detected with the first distal electrode pole (203); and determining (530) a second intrinsic atrioventricular conduction time between i) the intrinsic atrial contraction or the stimulation of the atrium and iii) the intrinsic ventricular contraction detected with the second distal electrode pole (223); d) setting (550) a stimulated atrioventricular conduction time for stimulating the ventricle (3, 5) of the heart (1) to be stimulated with the first distal electrode pole (203) and/or the second distal electrode pole (223), the stimulated atrioventricular conduction time being shorter than the shorter of the first intrinsic atrioventricular conduction time and the second intrinsic atrioventricular conduction time.

## Description

The present invention relates to an implantable medical device according to the preamble of claim 1 and to a method for operating such a device according to the preamble of claim 15.

Implantable medical devices for stimulating a human or animal heart can feature different functionalities. To give an example, a CRT-D device is designed and arranged to accomplish a cardiac resynchronization therapy and a defibrillation of the patient's heart. Such a CRT-D device typically has three electrodes, namely a combined right ventricular defibrillation and stimulation electrode, a right atrial stimulation and sensing electrode and a left ventricular coronary sinus electrode. Some manufacturers like BIOTRONIK also offer a more complex right ventricular electrode that integrates the atrial sensing functionality into the right ventricular stimulation electrode.

If the implantable medical device is designed and arranged as a CRT-P device, i.e., a device for cardiac resynchronization therapy and pacing (but no defibrillation), the general setup is almost identical to the previously described CRT-D device. However, the CRT-P device does not comprise a defibrillation electrode.

If the implantable medical device is designed and arranged as a device for employing a two-chamber therapy, it is also necessary to implant two distinct electrodes into the patient's heart. One electrode is guided into the right atrium, and the other is guided into the left ventricle. Both electrodes need to be connected with the stimulation generator, i.e., the implantable pulse generator. For this purpose, the implantable pulse generator typically comprises at least two connecting sockets.

As outlined above, prior art already teaches a specific variant of integrated electrodes that uses a proximal bipole for sensing electric signals in the patient's right atrium. Then, this variant of the ventricular electrode already takes over the functionality of the atrial electrode.

Regardless of the sensing and detecting functionalities of an implantable medical device are accomplished by one, two, or three electrodes, there remains the requirement of providing a patient with an optimum pacing adapted to the patient's health status.

It is an object of the present invention to provide an implantable medical device that enables a comprehensive cardiac resynchronization therapy.

This object is achieved with an implantable medical device for stimulating a human or animal heart having the features of claim 1.

Such an implantable medical device comprises a processor, a memory unit, a stimulation unit, and a detection unit. The stimulation unit is arranged and designed to stimulate a human or animal heart. The detection unit is designed and arranged to detect an electric signal of the same heart. In addition, the implantable medical device comprises a proximal electrode pole, a first distal electrode pole, and a second distal electrode pole. The proximal electrode pole, the first distal electrode pole, and the second distal electrode pole form part of the stimulation unit and of the detection unit.

According to an aspect of the presently claimed and described implantable medical device, the proximal electrode pole is configured to be implanted within an atrium of the heart to be stimulated. In addition, the first distal electrode pole is configured to be implanted within the septum of the heart to be stimulated. At this implantation site, the distal electrode pole is able to stimulate the left and the right ventricle of the heart to be stimulated, either simultaneously or individually, e.g., by left bundle branch area pacing (LBBAP). Thus, the first distal electrode pole is able to bypass a left and/or right bundle branch block and thus to achieve an efficient pacing of the left and/or right ventricle even in case that the physiologic stimulus lines are no longer working or no longer working correctly.

The second distal electrode pole is configured to be implanted within the apex (or within a different physiologic structure except the septum) of the right ventricle of the heart to be stimulated. Thus, the first distal electrode pole and the second distal electrode pole are configured to be implanted at different sites within the heart to be stimulated. Due to the first distal electrode pole and the second distal electrode pole, it is possible to detect a ventricular contraction of the heart to be stimulated at two different physiologic sites. With the first distal electrode pole that is to be implanted in the septum of the heart to be stimulated, a conduction signal of the right bundle branch can be typically detected. However, in some instances, no such signal can be observed. If the signal of the right bundle branch can be detected, it typically occurs earlier than a signal of a ventricular contraction detected by the second distal electrode pole to be implanted in the apex of the heart to be stimulated. If the first electrode pole is that deeply implanted within the septum, one can typically only detect the signal of the left bundle branch. In case of a left bundle branch block, no such signal can be observed at all. In such a case, the ventricular signal detected with the second distal electrode pole implanted within the apex of the heart to be stimulated is the earlier (or only) ventricular signal detected. Thus, the first distal electrode pole and the second distal electrode pole enable a detection of signals relating to the same ventricular contraction occurring at different time points.

The memory unit of the implantable medical device comprises a computer-readable program that causes the processor to perform the steps explained in the following when being executed on the processor.

In a first step, an intrinsic atrial contraction of the heart to be stimulated is detected with the proximal electrode pole. Alternatively, the atrium of the heart to be stimulated is stimulated with the proximal electrode pole to induce an atrial contraction.

In a further method step, an intrinsic ventricular contraction of the heart to be stimulated is detected with the first distal electrode pole and with the second distal electrode pole.

Subsequently, a first and a second intrinsic atrioventricular conduction time are determined. The first intrinsic atrioventricular conduction time is calculated between the intrinsic atrial contraction and the intrinsic ventricular contraction detected with the first distal electrode pole or between the stimulation of the atrium and the intrinsic ventricular contraction (being responsive to the stimulation of the atrium) detected with the first distal electrode pole. The second intrinsic atrioventricular conduction time is calculated between the intrinsic atrial contraction and the intrinsic ventricular contraction detected with the second distal electrode pole or between the stimulation of the atrium and the intrinsic ventricular contraction (being responsive to the stimulation of the atrium) detected with the second distal electrode pole. In doing so, factual measures reflecting the condition of the physiologic cardiac conduction system is obtained.

In a further method step, a stimulated atrioventricular conduction time is determined from the first and the second intrinsic atrioventricular conduction time and is set for subsequent ventricular simulations performed by the implantable medical device. This stimulated atrioventricular conduction time serves for triggering stimulation of a ventricle of the heart to be stimulated with the first distal electrode pole and/or with the second distal electrode pole. In this context, the stimulated atrioventricular conduction time is shorter than the shorter of the first intrinsic atrioventricular conduction time and the second intrinsic atrioventricular conduction time. Thus, the shorter value of the first atrioventricular conduction time and the second atrioventricular conduction time is used for setting the stimulated atrioventricular conduction time. However, the stimulated atrioventricular conduction time is made even shorter than the previously determined shortest intrinsic atrioventricular conduction time (which is either the first atrioventricular conduction time or the second atrioventricular conduction time).

By applying a stimulated atrioventricular conduction time that is shorter than the shortest observed intrinsic atrioventricular conduction time, it is guaranteed that a ventricular stimulation, in particular a left ventricular stimulation, safely occurs prior to any intrinsic excitation that might still be possible even in case of a left bundle branch block. Thus, a shortening of the stimulated atrioventricular conduction time with respect to the shortest intrinsic atrioventricular conduction time ensures a safe ventricular stimulation by the implantable medical device that affects both the right ventricle and the left ventricle of the heart to be stimulated. Consequently, an efficient cardiac resynchronization is achieved. This cardiac resynchronization resembles the physiologic needs of the patient much better than according to prior art solutions in which always the value of the intrinsic atrial ventricular conduction time detected with an apical electrode (i.e., an electrode implanted within the apex of the heart to be stimulated) is used for determining the stimulated atrioventricular conduction time. This leads to a stimulated atrioventricular conduction time that may be too long, in particular if a ventricular activity is also sensed with an electrode implanted within the septum of the heart to be stimulated.

In an embodiment, in case of any physiologic changes over time that change the intrinsic atrioventricular conduction time, the implantable medical device is configured to adapt the stimulated atrioventricular conduction time to the determined and amended intrinsic atrioventricular conduction time so that the stimulation provided by the implantable medical device is able to keep track of the condition of the heart to be stimulated and to reflect a highly physiologic stimulation.

In an embodiment, the proximal electrode pole is configured to be implanted within the right atrium, and the computer-readable program causes the processor to perform the following steps when being executed on the processor: a) detecting, with the proximal electrode pole, an intrinsic right atrial contraction of the heart to be stimulated or stimulating, with the proximal electrode pole, the right atrium of the heart to be stimulated; b) detecting, with the first distal electrode pole and with the second distal electrode pole, an intrinsic right ventricular contraction of the heart to be stimulated; c) determining the first intrinsic atrioventricular conduction time between i) the intrinsic right atrial contraction or the stimulation of the right atrium and ii) the intrinsic right ventricular contraction detected with the first distal electrode pole; and determining the second intrinsic atrioventricular conduction time between i) the intrinsic right atrial contraction or the stimulation of the right atrium and iii) the intrinsic right ventricular contraction detected with the second distal electrode pole; d) setting the stimulated atrioventricular conduction time for stimulating the left ventricle of the heart to be stimulated with the first distal electrode pole and/or the second distal electrode pole, the stimulated atrioventricular conduction time being shorter than the shorter of the first intrinsic atrioventricular conduction time and the second intrinsic atrioventricular conduction time. At its intended implantation site, the first distal electrode pole is able to stimulate at least the left ventricle of the patient's heart by left bundle branch area pacing (LBBAP). Thus, the first distal electrode pole is able to bypass a left bundle branch block and thus to achieve an efficient pacing of the left ventricle even in case that the physiologic stimulus lines are no longer working or no longer working correctly. The second distal electrode pole is, when implanted within the apex of the heart to be stimulated, able to stimulate the right ventricle of the heart to be stimulated and serves for backup stimulation.

In an embodiment, the proximal electrode pole is part of an atrial electrode which is implanted in the right atrium. The atrial electrode may be fixed to an atrial wall of the right atrium at its distal terminus. Moreover, the first distal electrode pole may be part of a first ventricular electrode which is implanted in the right ventricle. The first ventricular electrode may be fixed in the septum of the heart. In addition, the second distal electrode pole may be part of a second ventricular electrode which is implanted in the right ventricle. The second ventricular electrode may be fixed in the apex of the heart. Thus, this embodiment makes use of three distinct electrodes.

In an embodiment, the proximal electrode pole and the first distal electrode pole are part of a first electrode that is implanted in the right atrium and the right ventricle and fixed in the septum of the heart. In this embodiment, the proximal electrode pole is not fixed to the atrial wall, but may be floating within the right atrium.

In an embodiment, the proximal electrode pole and the second distal electrode pole are part of a second electrode that is implanted in the right atrium and the right ventricle and fixed in the apex of the heart. Also in this embodiment, the proximal electrode pole is not fixed to the atrial wall, but may be floating within the right atrium.

In an embodiment, the proximal electrode pole is a single electrode pole. For sensing atrial signals and/or for stimulating the atrium of the heart to be stimulated, a housing of the implantable medical device can be used as counter electrode pole in this embodiment.

In an embodiment, the proximal electrode pole is a bipole. This proximal bipole is arranged and designed to detect an intrinsic atrial signal of the heart to be stimulated. After implantation of the electrode of the implantable medical device on which the proximal bipole is arranged, the proximal bipole is located within the right atrium so that the intrinsic atrial signal sensed by the proximal bipole can then be used to trigger the further stimulation pulses in order to deliver the LBBAP stimulation to the ventricle of the heart. In case of such a proximal bipole, it is not necessary to use a housing of the implantable medical device as counter electrode pole. Rather, one of the electrode poles of the proximal bipole can serve as counter electrode pole for the respective other electrode pole of the proximal bipole.

In an embodiment, the proximal bipole comprises two ring electrodes spaced apart from each other serving as electrode pole and counter electrode pole, respectively. The two ring electrodes may be floatingly arranged within the atrium.

In another embodiment, the proximal bipole comprises a tip electrode pole and a ring electrode serving as electrode pole and counter electrode pole. The tip electrode pole may have a helix shape and may be configured to be secured in cardiac tissue.

In an embodiment, the first ventricular electrode and/or the second ventricular electrode comprises a helix at its distal end. This helix is designed and configured to be secured within cardiac tissue. For this purpose, the helix can be turned into the cardiac tissue, e.g., into the septum or the apex of the patient's heart. After having implanted the first and/or second ventricular electrode into the cardiac tissue like the septum, in particular into the deep septum, it is possible to achieve an effective stimulation of the left ventricle even if no electrode is directly placed within the left ventricle or on an outside thereof (as in case of prior art left ventricular stimulation electrodes). An implantation of the first distal electrode pole in the deep septum at a position distally of a left bundle branch block enables left bundle branch area pacing without requiring a separate left ventricular electrode.

In an embodiment, the helix is designed as fixed fixing helix. In another embodiment, the helix is designed as unscrewable fixing helix. Either design is particularly appropriate for fixing the first and/or second ventricular electrode within the septum or apex of the patient's heart.

In an embodiment, the first distal electrode pole and/or the second distal electrode pole is a single electrode pole. For sensing and stimulation functionalities, a housing of the implantable medical device is then used as counter electrode pole for the respective single distal electrode pole.

In an embodiment, the first distal electrode pole and/or the second distal electrode pole is a distal bipole. This distal bipole is arranged and designed to detect an intrinsic right ventricular signal of the heart to be stimulated. After implantation of the first ventricular electrode and/or the second ventricular electrode, the distal bipole is located within the septum of the heart to be stimulated or within the apex of the heart to be stimulated. If the septum is chosen as implantation site, the distal bipole is well suited to provide stimulation pulses for LBBAP, as described above for a single distal electrode pole. In case of such a distal bipole, it is not necessary to use a housing of the implantable medical device as counter electrode pole. Rather, one of the electrode poles of the distal bipole can serve as counter electrode pole for the respective other electrode pole of the distal bipole.

In an embodiment, the above-mentioned helix forms at least a part of the first distal bipole and/or the second distal bipole. Expressed in other words, at least one electrode pole of the first distal bipole or of the second distal bipole is realized by the respective helix that is also used to secure the first ventricular electrode or second ventricular electrode within the septum or the apex of the patient's heart. This guarantees a very efficient energy transfer from the first ventricular electrode or the second ventricular electrode into the surrounding cardiac tissue.

In an embodiment, the other electrode pole of the first distal bipole and/or the second distal bipole may be a ring electrode or a shock coil.

The first distal bipole comprises a first electrode pole and a second electrode pole located proximally from the first electrode pole. Likewise, the second distal bipole comprises a third electrode pole and a fourth electrode pole located proximally from the third electrode pole.

In an embodiment, a distance between a distal end of the second electrode pole and a proximal end of the first electrode pole (and/or a distance between a distal end of the fourth electrode pole and a proximal end of the third electrode pole) lies in a range of from 1 mm to 30 mm, in particular from 2 mm to 25 mm, in particular from 3 mm to 20 mm, in particular from 4 mm to 15 mm, in particular from 5 mm to 10 mm. Such a distance between the individual electrode poles is particularly appropriate to allow a stimulation of different cardiac regions by the individual electrode poles after the first ventricular electrode and/or the second ventricular electrode has been implanted into the septum or apex of the patient's heart. E.g., the first electrode pole can stimulate the left bundle branch, i.e., it can perform left bundle branch area pacing (LBBAP). Likewise, the second electrode pole can then stimulate the right bundle branch, i.e., it can perform right bundle branch area pacing (RBBAP). In addition, the second electrode pole can particularly well detect right ventricular signals in this position. The third and fourth electrode pole can stimulate different apical regions and can well detect right ventricular signals within the apex of the patient's heart.

In an embodiment, the computer-readable program causes the processor to subtract a predeterminable absolute value from the determined shorter intrinsic atrioventricular conduction time for determining the stimulated atrioventricular conduction time that is then set to be used for further stimulation events. In an embodiment, the absolute amount to be subtracted lies in a range of from 1 ms to 100 ms, in particular from 2 ms to 95 ms, in particular from 3 ms to 90 ms, in particular from 4 ms to 85 ms, in particular from 5 ms to 80 ms, in particular from 6 ms to 75 ms, in particular from 7 ms to 70 ms, in particular from 8 ms to 65 ms, in particular from 8 ms to 60 ms, in particular from 9 ms to 55 ms, in particular from 10 ms to 50 ms, in particular from 15 ms to 45 ms, in particular from 20 ms to 40 ms, in particular from 25 ms to 35 ms.

In an embodiment, the computer-readable program causes the processor to subtract a predeterminable relative value from the determined shorter intrinsic atrioventricular conduction time for defining the stimulated atrioventricular conduction time that is set for subsequent stimulation events performed by the implantable medical device. In an embodiment, the relative value to be subtracted lies in a range of from 1 % to 50 %, in particular from 2 % to 45 %, in particular from 3 % to 40 %, in particular from 4 % to 35 %, in particular from 5 % to 30 %, in particular from 6 % to 25 %, in particular from 7 % to 20 %, in particular from 8 % to 15 %, in particular from 9 % to 10 %. A subtraction of such a relative value can adjust the shorter intrinsic atrioventricular conduction time to result in the stimulated atrioventricular conduction time in an even more physiologic way than the subtraction of the absolute value typically is able to do.

In an embodiment, the computer-readable program causes the processor to regularly repeat the steps of a) detecting an intrinsic atrial contraction or stimulating the right atrium, b) detecting an intrinsic right ventricular contraction of the heart, c) determining the first and second intrinsic atrioventricular conduction time, and d) setting the stimulated atrioventricular conduction time after a predeterminable number of cardiac cycles and/or after a predeterminable time interval. In doing so, a continuous adaptation of the stimulated atrioventricular conduction time to a possibly changing intrinsic atrioventricular conduction time can be achieved in a highly efficient manner. Such a regularly repetition can also be denoted as cyclic measuring.

In an embodiment, repetition of the precedingly explained method steps is performed after a predeterminable number of cardiac cycles, wherein the predeterminable number lies in a range of from 10 to 1000, in particular from 20 to 900, in particular from 30 to 800, in particular from 40 to 700, in particular from 50 to 600, in particular from 60 to 500, in particular from 70 to 400, in particular from 80 to 300, in particular from 90 to 200, in particular from 100 to 150.

In an embodiment, the repetition takes place after a predeterminable time period has passed, wherein the predeterminable time period lies in a range of from 10 seconds to 1000 seconds, in particular from 20 seconds to 900 seconds, in particular from 30 seconds to 800 seconds, in particular from 40 seconds to 700 seconds, in particular from 50 seconds to 600 seconds, in particular from 60 seconds to 500 seconds, in particular from 70 second to 400 seconds, in particular from 80 seconds to 300 seconds, in particular from 90 seconds to 200 seconds, in particular from 100 seconds to 150 seconds.

In an embodiment, the computer-readable program causes the processor to increase the stimulated atrioventricular conduction time to an amount that is longer than an expected intrinsic atrioventricular conduction time when the step of determining the intrinsic atrioventricular conduction time is to be performed. Such an increase of the stimulated atrioventricular conduction time results in a stimulated atrioventricular conduction time that is longer than the first and second intrinsic atrioventricular conduction time. Consequently, a stimulation under application of the stimulated atrioventricular conduction time will not result in a cardiac contraction (since the heart is still in its refractory phase) or will at least not disturb a physiologic intrinsic ventricular contraction so that an updated value of the first and second intrinsic atrioventricular conduction time can be easily recorded and used for defining an updated value of the stimulated atrioventricular conduction time.

In an embodiment, the computer-readable program causes the processor to detect the intrinsic ventricular contraction, in particular the intrinsic right ventricular contraction, by evaluating a far-field electrocardiogram that is measured between i) the first distal electrode pole or the second distal electrode pole and ii) a housing of the implantable medical device. Such an evaluation of the far-field electrocardiogram is a particularly appropriate possibility to detect the intrinsic cardiac activity with a single first distal electrode pole and/or a single second distal electrode pole. Thus, when relying on the evaluation of the far-field electrocardiogram, it is not necessary to provide another electrode pole for the first and/or the second distal electrode pole. This reduces the amount of electrode leads to be guided within the electrodes and thus reduces the complexity of the electrodes of the implantable medical device.

In an embodiment, the implantable medical device comprises a shock coil that is located proximally of the first distal electrode pole or proximally of the second distal electrode pole. Such a shock coil can well be used for providing a defibrillation shock to the heart to be stimulated. Then, the implantable medical device can be used as CRT-D device. In this embodiment, the computer-readable program causes the processor to detect the intrinsic ventricular contraction, in particular the intrinsic right ventricular contraction, by evaluating a far-field electrocardiogram that is measured between the shock coil and a housing of the implantable medical device. A far-field electrocardiogram measured between the shock coil and the housing of the implantable medical device may comprise stronger signals than a far-field electrocardiogram measured between the distal electrode pole and the housing of the implantable medical device.

In an embodiment, the shock coil has a surface of at least 150 mm², in particular at least 175 mm², in particular at least 200 mm², in particular at least 225 mm², in particular at least 250 mm². Such a surface enables a sufficiently big shock pulse to be delivered by the shock coil to achieve an efficient cardiac defibrillation of the patient's heart.

In an embodiment, the computer-readable program causes the processor to use an earliest time point of a ventricular excitation, in particular of a right ventricular excitation, as a measure for the intrinsic ventricular contraction, in particular for the intrinsic right ventricular contraction. Typically, this earliest time point of a ventricular excitation is the very beginning of the so-called QRS complex in an electrocardiogram. This QRS complex represents a ventricular excitation during a cardiac cycle. The beginning of the QRS complex as indication of the time point of the intrinsic ventricular contraction can be used both in case of evaluating a regular electrocardiogram (measured between two electrode poles that are both located on one of the ventricular electrodes) and in case of evaluating a far-field electrocardiogram (measured between an electrode pole located on one of the ventricular electrodes and a housing of the implantable medical device). The beginning of the QRS complex is a particularly appropriate time point for defining the start of the intrinsic ventricular contraction that is used for determining the intrinsic atrioventricular conduction time.

In an embodiment, the determination of the earliest time point of the ventricular excitation, in particular of the right ventricular excitation, is done via a morphologic signal evaluation. To give an example, the slope of a signal curve (also referred to as signal rise speed) in combination with a minimum value of the amplitude is a particularly appropriate morphologic measure to identify the earliest time point of the ventricular excitation from a measured signal curve like an electrocardiogram. As outlined above, the electrocardiogram can be a regular electrocardiogram or a far-field electrocardiogram.

In an embodiment, the computer-readable program causes the processor to perform the step of setting the stimulated atrioventricular conduction time only if at least one of the first determined intrinsic atrioventricular conduction time and the second determined intrinsic atrioventricular conduction time lies within a predeterminable range. In case that both the first intrinsic atrioventricular conduction time and the second determined intrinsic atrioventricular conduction time lie outside the predeterminable range, the stimulated atrioventricular conduction time is set to a predeterminable fixed value. This embodiment prevents the setting of a non-physiologic stimulated atrioventricular conduction time in case that the determined first and/or second intrinsic atrioventricular conduction time was calculated from an atypic cardiac cycle such as a cardiac cycle comprising an atrial extrasystole. Thus, this embodiment increases the safety of the implantable medical device and guarantees a high user-friendliness of the implantable medical device.

In an embodiment, the predeterminable range of the atrioventricular conduction time is a range of from 0.10 s to 0.25 s, in particular from 0.12 s to 0.22 s, in particular from 0.13 s to 0.20 s, in particular from 0.14 s to 0.18 s.

In an embodiment, the computer-readable program causes the processor to set the stimulated atrioventricular conduction time to a value lying within a predeterminable range. This embodiment increases the safety of the implantable medical device, too. It ensures that only physiologically sensible stimulated atrioventricular conduction times are applied by the implantable medical device during its operation.

In an embodiment, the allowable predeterminable range of the stimulated atrioventricular conduction time is a range of from 0.05 s to 0.20 s, in particular from 0.10 s to 0.15 s, in particular from 0.11 s to 0.12 s, in particular from 0.12 s to 0.13 s.

In an aspect, the present invention relates to a method for operating an implantable medical device according to the preceding explanations. This method comprises the steps explained in the following.

In a first step, an intrinsic atrial contraction of the heart to be stimulated is detected with the proximal electrode pole.

In a further method step, an intrinsic ventricular contraction of the heart to be stimulated is detected with the first distal electrode pole and the second distal electrode pole.

Subsequently, a first and a second intrinsic atrioventricular conduction time are determined. The first atrioventricular conduction time is calculated between the intrinsic atrial contraction and the intrinsic ventricular contraction detected with the first distal electrode pole. The second atrioventricular conduction time is calculated between the intrinsic atrial contraction and the intrinsic ventricular contraction detected with the second distal electrode pole. In doing so, factual measures reflecting the condition of the physiologic cardiac conduction system are obtained.

In a further method step, a stimulated atrioventricular conduction time is determined from the shorter of the first and second intrinsic atrioventricular conduction time and is set for subsequent ventricular simulations performed by the implantable medical device. This stimulated atrioventricular conduction time serves for triggering a stimulation of a ventricle of the heart to be stimulated with the distal electrode pole. In this context, the stimulated atrioventricular conduction time is shorter than the shorter of the first intrinsic atrioventricular conduction time and the second intrinsic atrioventricular conduction time. By applying a stimulated atrioventricular conduction time that is shorter than the shorter determined intrinsic atrioventricular conduction time, it is guaranteed that a ventricular stimulation, in particular a left ventricular stimulation, safely occurs prior to any intrinsic excitation that might still be possible even in case of a left and/or right bundle branch block.

In an embodiment, the method for operating an implantable medical device comprises the following steps: a) detecting, with the proximal electrode pole, an intrinsic right atrial contraction of the heart to be stimulated or stimulating, with the proximal electrode pole, the atrium of the heart to be stimulated; b) detecting, with the first distal electrode pole and the second distal electrode pole, an intrinsic right ventricular contraction of the heart to be stimulated; c) determining the first intrinsic atrioventricular conduction time between the intrinsic right atrial contraction or the stimulation of the atrium and the intrinsic right ventricular contraction detected with the first distal electrode pole; and determining the second intrinsic atrioventricular conduction time between the intrinsic right atrial contraction or the stimulation of the atrium and the intrinsic right ventricular contraction detected with the second distal electrode pole; d) setting a stimulated atrioventricular conduction time for stimulating the left ventricle of the heart to be stimulated with the first and/or second distal electrode pole, the stimulated atrioventricular conduction time being shorter than the shorter of the first intrinsic atrioventricular conduction time and the second intrinsic atrioventricular conduction time.

In an aspect, the present invention relates to a medical method for providing a cardiac resynchronization therapy to a patient in need thereof. This method comprises the steps explained in the following.

In a first step, an intrinsic atrial contraction of the heart to be stimulated is detected with a proximal electrode pole of an electrode of an implantable medical device for stimulating a human or animal heart. Alternatively, an atrium of the heart to be stimulated is stimulated with the proximal electrode pole to induce an atrial contraction. An implantable medical device according to the preceding explanations is particularly appropriate for carrying out this method.

In a further method step, an intrinsic ventricular contraction of the heart to be stimulated is detected with a first distal electrode pole and with a second distal electrode pole. In this context, the first distal electrode pole is implanted within the septum of the patient's heart and the second distal electrode pole is implanted within the apex of the patient's heart.

Subsequently, a first and a second intrinsic atrioventricular conduction time are determined. The first atrioventricular conduction time is calculated between the intrinsic atrial contraction and the intrinsic ventricular contraction detected with the first distal electrode pole or between the stimulation of the atrium and the intrinsic ventricular contraction (being responsive to the stimulation of the atrium) detected with the first distal electrode pole. The second atrioventricular conduction time is calculated between the intrinsic atrial contraction and the intrinsic ventricular contraction detected with the second distal electrode pole or between the stimulation of the atrium and the intrinsic ventricular contraction (being responsive to the stimulation of the atrium) detected with the second distal electrode pole. In doing so, factual measures reflecting the condition of the physiologic cardiac conduction system are obtained.

In a further method step, a stimulated atrioventricular conduction time is determined from the shorter of the first intrinsic atrioventricular conduction time and the second intrinsic atrioventricular conduction time. This stimulated atrioventricular conduction time is set for subsequent ventricular simulations performed by the implantable medical device. This stimulated atrioventricular conduction time serves for triggering a stimulation of a ventricle of the patient's heart with the distal electrode pole. In this context, the stimulated atrioventricular conduction time is shorter than the shorter of the first intrinsic atrioventricular conduction time and the second intrinsic atrioventricular conduction time.

Finally, the ventricle of the patient's heart is stimulated with at least one stimulation pulse emitted by the first distal electrode pole and/or the second distal electrode pole upon expiration of the stimulated atrioventricular conduction time. This at least one stimulation pulse serves for efficient cardiac resynchronization of the patient's heart.

In a further embodiment, the method for providing a cardiac resynchronization comprises, in particular, the following steps: a) detecting, with the proximal electrode pole, an intrinsic right atrial contraction of the heart to be stimulated or stimulating, with the proximal electrode pole, the right atrium of the heart to be stimulated, wherein the proximal electrode pole is implanted within the right atrium of the patient's heart; b) detecting, with the first distal electrode pole and with the second distal electrode pole, an intrinsic right ventricular contraction of the heart to be stimulated, wherein the first distal electrode pole is implanted within the septum of the patient's heart and wherein the second distal electrode pole is implanted within the apex of the patient's heart; c) determining the first intrinsic atrioventricular conduction time between i) the intrinsic right atrial contraction or the stimulation of the right atrium and ii) the intrinsic right ventricular contraction detected with the first distal electrode pole; and determining the second intrinsic atrioventricular conduction time between i) the intrinsic right atrial contraction or the stimulation of the right atrium and iii) the intrinsic right ventricular contraction detected with the second distal electrode pole; d) setting the stimulated atrioventricular conduction time for stimulating the left ventricle of the patient's heart, the stimulated atrioventricular conduction time being shorter than the shorter of the first intrinsic atrioventricular conduction time and the second intrinsic atrioventricular conduction time; e) stimulating the left ventricle of the patient's heart with the first distal electrode pole and/or with the second distal electrode pole.

All embodiments of the implantable medical device can be combined in any desired way and can be transferred either individually or in any arbitrary combination to each of the methods. Likewise, all embodiments of each of the methods can be combined in any desired way and can be transferred either individually or in any arbitrary combination to the implantable medical device and to the respective other method.

Further details of aspects of the present invention will be explained in the following making reference to exemplary embodiments and accompanying Figures. In the Figures:
- Figure 1: shows a first embodiment of an implantable medical device implanted into a human heart;
- Figure 2: shows a second embodiment of an implantable medical device implanted into a human heart;
- Figure 3: shows a third embodiment of an implantable medical device implanted into a human heart;
- Figure 4: shows a fourth embodiment of an implantable medical device implanted into a human heart;
- Figure 5: schematically shows different components of an embodiment of an implantable medical device; and
- Figure 6: shows a schematic flowchart of an embodiment of a method performed by an implantable medical device during operation.

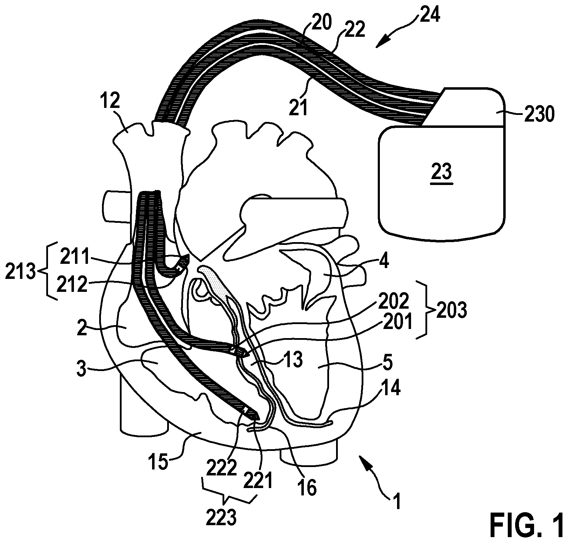

Figure 1 shows a human heart 1 into which a first ventricular electrode 20, an atrial electrode 21, and a second ventricular electrode 22 are implanted. The first ventricular electrode 20, the atrial electrode 21, and the second ventricular electrode 22 are guided through the superior vena cava 12 into the right atrium 2. The first ventricular electrode 20 is furthermore guided into the right ventricle 3 and fixed within the septum 13 separating the right ventricle 3 from the left ventricle 5. The first ventricular electrode 20 is implanted in a deep septal position so that it can stimulate the left bundle branch 14 of the human heart 1 and thus stimulate the left ventricle 5 even though it does not directly contact the left ventricle 5. The second ventricular electrode 22 is also guided into the right ventricle 3 but fixed within the apex 15 of the heart 1.

The atrial electrode 21 serves for detecting atrial signals and/or stimulating atrial tissue. For this purpose, the atrial electrode 21 comprises a first atrial electrode pole 211 and a second atrial electrode pole 212 that is located proximally of the first distal atrial electrode pole 211. The first atrial electrode pole 211 and the second atrial pole 212 form an atrial bipole 213 that is at least partially implanted into atrial tissue. This atrial bipole 213 represents a proximal electrode pole.

The first ventricular electrode 20 comprises a first distal electrode pole 201 and a second distal electrode pole 202 that is located proximally of the first distal electrode pole 201. The first distal electrode pole 201 and the second distal electrode pole 202 form a first distal bipole 203 that represents a distal electrode pole. The first distal bipole 203 is fixed within the septum 13 of the patient's heart 1.

The second ventricular electrode 22 comprises a third distal electrode pole 221 and a fourth distal electrode pole 222 that is located proximally of the third distal electrode pole 221. The third distal electrode pole 221 and the fourth distal electrode pole 222 form a second distal bipole 223 that represents a distal electrode pole. The first distal bipole 203 is fixed within the apex 15 of the patient's heart 1.

The first ventricular electrode 20, the atrial electrode 21, and the second ventricular electrode 22 form part of a CRT-P device 24 that represents an implantable medical device. The CRT-P device 24 comprises a stimulation generator 23 (also referred to as housing) that comprises a header 230. The first ventricular electrode 20, the atrial electrode 21, and the second ventricular electrode 22 are plugged with their electrode connectors into electrode connector receiving sockets arranged in this header 230.

The first distal electrode pole 203 is implanted in the septum 13 between the left bundle branch 14 and the right bundle branch 16 of the patient's heart 1. Most patients have an intact right bundle branch 16 so that the intrinsic atrioventricular conduction time can be measured between the atrial bipole 213 and the first distal electrode pole 203 as well as between the atrial bipole 213 and the second distal electrode pole 223. Typically, the first intrinsic atrioventricular conduction time measured between the atrial bipole 213 and the first distal electrode pole 203 is shorter than the second intrinsic atrioventricular conduction time measured between the atrial bipole 213 and the second distal electrode pole 223. However, if the conduction along the right bundle branch 16 is disturbed or if the first distal electrode pole 203 is implanted at a site within the septum 13 at which it cannot well sense signals extending along the right bundle branch 16, the second intrinsic atrioventricular conduction time measured between the atrial bipole 213 and the second distal electrode pole 223 is shorter than the first intrinsic conduction time (which cannot be measured at all or which is longer due to conduction delays).

The CRT-P device 24 features a functionality according to which the shorter of the first intrinsic atrioventricular conduction time and the second intrinsic atrioventricular conduction time is chosen to determine the stimulated atrioventricular conduction time for subsequent ventricular stimulation carried out by the CRT-P device 24 with the first distal electrode pole 203 and/or the second distal electrode pole 223.

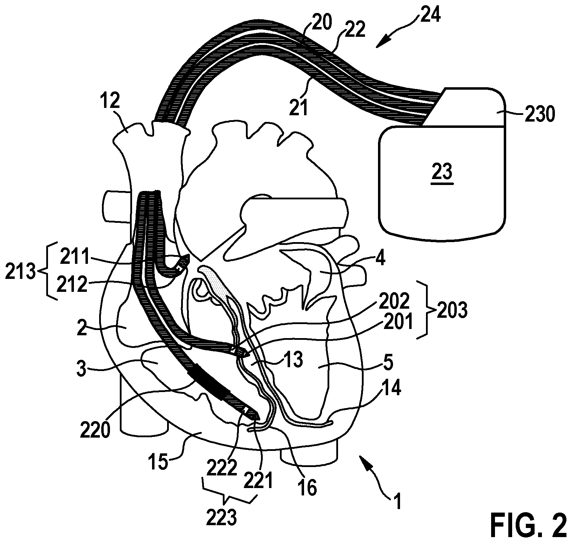

Figure 2 shows an embodiment of a CRT-D device 24 that is very similar to the CRT-P device 24 shown in Figure 1. In this and in all following Figures, similar elements will be denoted with the same numeral reference. The CRT-D device 24 additionally comprises a shock coil 220 located on the second ventricular electrode 22 proximally from the second distal electrode pole 223. This shock coil 220 enables the provision of a defibrillation shock to the heart 1 in case that not only a resynchronization, but also a defibrillation is required. Thus, the second ventricular electrode 22 of the embodiment shown in Figure 2 is able to provide a CRT-D therapy. Expressed in other words, this second ventricular electrode 22 forms part of a CRT-D system 24 as example of an implantable medical device.

The shock coil 220 can also be used to measure a far-field electrocardiogram between the shock coil 220 and the stimulation generator 23. Thus, the embodiment shown in Figure 2 offers the possibility of measuring both near-field electrocardiograms (between the individual electrode poles of the second distal bipole 223 or the atrial bipole 213) as well as between the shock coil 220 and the stimulation generator 23. In addition, it is possible to use either of the first distal electrode pole 201, the second distal electrode pole 202, the first atrial electrode pole 211, the second atrial electrode pole 212, the third distal electrode pole 221, and the fourth distal electrode pole 222, for measuring a far-field electrocardiogram against the stimulation generator 23 of the CRT-D device 24.

In other, not illustrated embodiments, the shock coil 220 is not located on the second ventricular electrode 22, but rather on the first ventricular electrode 20.

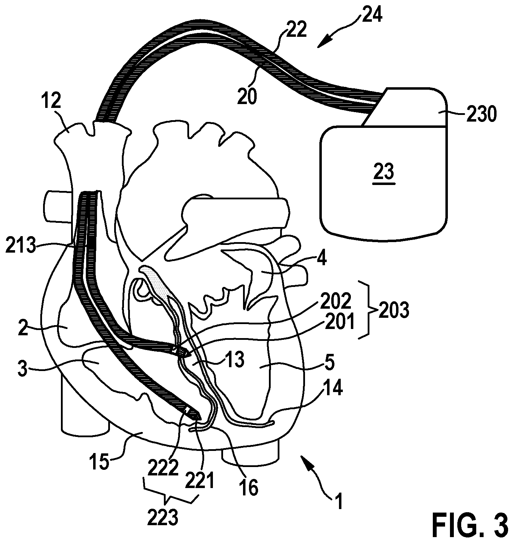

Figure 3 shows again an embodiment of a CRT-P device 24 implanted into a human heart 1 that is very similar to the embodiment shown in Figure 1. The CRT-P device 24 of Figure 3 does, however, not comprise an atrial electrode 21. Rather, the first ventricular electrode 20 features also the functionalities of the atrial electrode of the preceding explained embodiments. For this purpose, the first ventricular electrode 20 comprises an atrial electrode pole 213 that serves as proximal electrode pole. It is located on the first ventricular electrode 20 such that it is placed in a floating position within the right atrium 2 after implantation of the CRT-P device 24. The atrial electrode pole 213 serves for detecting atrial signals and/or stimulating atrial tissue.

During operation of the CRT-P device 24, a far-field electrocardiogram is measured between the distal electrode pole 203 and the stimulation generator 23 and/or between the atrial electrode pole 213 and the stimulation generator 23.

The first ventricular electrode 20 comprises a first distal electrode pole 203 having the shape of a helix and being fixed within the septum 13 of the patient's heart 1. The first ventricular electrode 20 is implanted in a deep septal position so that it can stimulate the left bundle branch 14 of the human heart 1 and thus stimulate the left ventricle 5 even though it does not directly contact the left ventricle 5 (nor the left atrium 4).

The second ventricular electrode 22 is - as in case of the embodiment shown in Figure 1 - implanted within the apex 15 of the patient's heart 1 so that the second distal electrode pole 223 can stimulate the apical tissue.

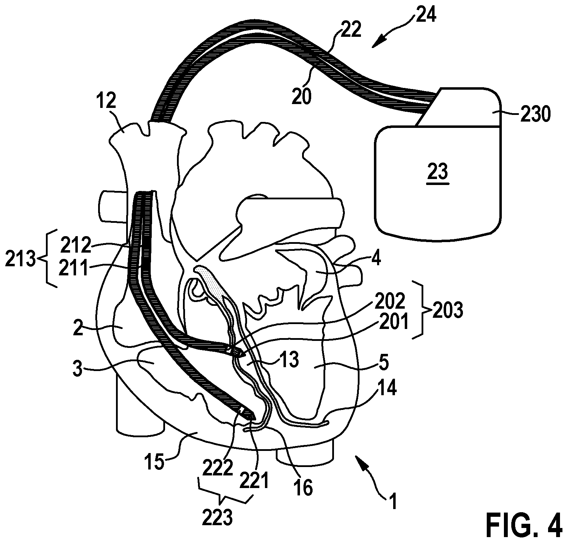

Figure 4 shows another embodiment of a CRT-P device 24 implanted into a human heart 1 that is very similar to the embodiment shown in Figure 3.

In contrast to the embodiment shown in Figure 3, the CRT-P device 24 of Figure 3 has a first ventricular electrode 20 that comprises a first distal bipole 203 and an atrial bipole 213 that is designed as floating atrial bipole 213.

The first distal bipole 203 comprises a first distal electrode pole 201 and a second distal electrode pole 202 that is located proximally of the first distal electrode pole 201. The distal bipole 203 is fixed within the septum 13 of the patient's heart 1 by means of a helix that forms the first distal electrode pole 201 of the distal bipole 203.

The atrial electrode pole 213 of the biventricular electrode 20 is designed as atrial bipole 213. It comprises a first atrial electrode pole 211 and a second atrial electrode pole 212 that is located proximally of the first distal atrial electrode pole 211.

Instead of measuring a far-field electrocardiogram between the distal electrode pole 203 and the stimulation generator 23 and/or between the atrial electrode pole 213 and the stimulation generator 23 like in case of the embodiment shown in Figure 3, the distal bipole 203 and the atrial bipole 213 enable a measurement of a near-field electrocardiogram between the first distal electrode pole 201 and the second distal electrode pole 202 on the one hand and between the first atrial electrode pole 211 and the second electrode pole 212 on the other hand.

The second ventricular electrode 22 is - as in case of the embodiments shown in Figures 1, 2 and 3 - implanted within the apex 15 of the patient's heart 1 so that the second distal electrode pole 223 can stimulate the apical tissue.

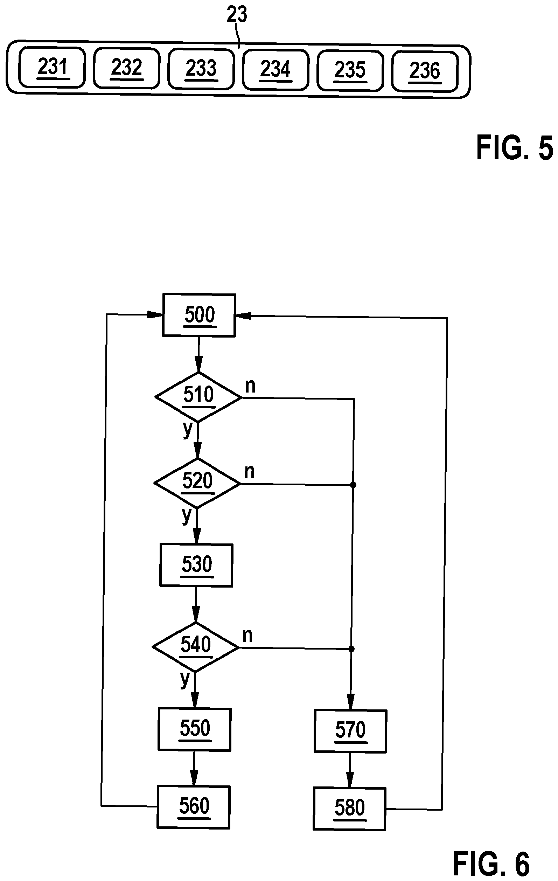

Figure 5 schematically illustrates individual components of an embodiment of an implantable medical device, such as of the embodiments shown in Figures 1 to 4, that are comprised within the stimulation generator 23 of the implantable medical device. The stimulation generator 23 houses a detection unit 231 (also referred to as sensing unit) that typically comprises an analog-to-digital converter, a bandpass filter, and an offset compensation. The detection unit 231 is operatively connected with a processor 232 that has access to a memory unit 233. The memory unit 233 serves for storing instructions for the processor 232 as well as data detected by the detection unit 231. The stimulation generator 23 further optionally comprises an evaluation unit 234 that can also be part of the processor 232 and that serves for extracting features from the detected cardiac electric signal. The stimulation generator 23 further comprises a stimulation unit 235 that serves for stimulating the heart from which the detection unit 231 detects electric signals. The first ventricular electrode 20 (along with its electrode poles 203 and optionally 213; confer Figures 1 to 4) and the second ventricular electrode 22 (along with its electrode pole 223; confer figures 1 to 4) forms part of the detection unit 231 and of the stimulation unit 235. Additionally, the stimulation generator 23 comprises a communication unit 236 that serves for data transfer to a (remote) programming device.

Figure 6 shows a schematic flowchart of a cyclic adaptation of the stimulated atrioventricular conduction time that is performed in an embodiment of the presently claimed and described implantable medical device, such as the implantable CRT-P device 24 of Figures 1, 3 and 4 or the implantable CRT-D device 24 of Figure 2. The method depicted in Figure 6 will now be explained in more detail making also references to Figures 1 to 4.

In an atrial sensing/atrial pacing step 500, an intrinsic atrial contraction of the heart 1 is detected with the proximal electrode pole 213 of the atrial electrode 21 or the first ventricular electrode 20 of the CRT-P device 24 or the CRT-D device 24 (confer Figures 1 to 4 for more details on the devices). Alternatively, the proximal electrode pole 213 is used for stimulating the right atrium 2 of the patient's heart 1 in this atrial sensing/atrial pacing step 500.

In a subsequent first decision step 510 it is determined whether the detected atrial rate is within a predetermined limit. If this is the case (indicated by a "y" meaning "yes"), the method will proceed to a second decision step 520. If this is not the case (indicated by an "n" meaning "no"), the stimulated atrioventricular conduction time to be applied by the implantable CRT-P/CRT-D device 24 is set to a programmed atrioventricular delay in a setting step 570. The CRT-P/CRT-D device 24 will then proceed with pacing for a predeterminable number of cardiac cycles (such as 60 cycles) in a pacing step 580 applying the programmed atrioventricular delay. Afterwards, the method will return to the initial atrial sensing/atrial pacing step 500.

Assuming that the first decision step 510 resulted in an atrial rate that was within the predeterminable limit, it will be determined in the second decision step 520 whether the sensed atrial signal is a regular atrial signal (y) or its to be considered as atrial extrasystole (n). In case of a detected (or suspected) atrial extrasystole, the method will proceed with the setting step 570 as explained above. If, however, the sensed atrial signal is considered to be a regular atrial signal, the method will proceed to an atrioventricular delay measuring step 530.

In this atrioventricular delay measuring step 530 an intrinsic atrioventricular conduction time (also referred to as atrioventricular delay) is determined. For this purpose, a non-physiologic long stimulated atrioventricular delay is set. Optionally, a ventricular trigger signal is suspended. Consequently, the pacing by the CRT-P/CRT-D device 24 will not occur at all or will only occur after an intrinsic (right) ventricular contraction. This enables measuring a first intrinsic atrioventricular conduction time between i) the intrinsic atrial contraction or the stimulation of the atrium and ii) the intrinsic ventricular contraction detected with the first distal electrode pole 203. Likewise, this enables measuring a second intrinsic atrioventricular conduction time between i) the intrinsic atrial contraction or the stimulation of the atrium and iii) the intrinsic ventricular contraction detected with the second distal electrode pole 223.

In a third decision step 540, it is determined whether the measured intrinsic atrioventricular conduction times lie within a predeterminable time limit. If this is not the case (n), the method will proceed with the setting step 570 as indicated above. If, however, the measured intrinsic atrioventricular conduction times lie within the predeterminable time limit (y) the method proceeds to an adjustment step 550 in which the stimulated atrioventricular delay is set on the basis of the determined intrinsic atrioventricular delay. In this context, the stimulated atrioventricular delay is set to be shorter than the shorter value of the determined first intrinsic atrioventricular delay and the determined second intrinsic atrioventricular delay. In doing so, it is ensured that the stimulation provided by the CRT-P/CRT-D device 24 is provided earlier than an intrinsic ventricular contraction would occur. Consequently, the provided stimulation will result in a synchronous contraction of the right ventricle 3 and the left ventricle 5 of the patient's heart 1.

The stimulation with the stimulated atrioventricular delay that was set in the adjustment step 550 is performed in a pacing step 560 for a predeterminable number of cardiac cycles, e.g., for 60 cycles. Afterwards, the method returns to the atrial sensing/atrial pacing step 500. Then, a re-adjustment of the stimulated atrioventricular delay will be performed so that any physiologic changes of the intrinsic atrioventricular conduction time will be reflected in the stimulated atrioventricular conduction time in a highly timely manner.

## Claims

1. Implantable medical device for stimulating a human or animal heart, comprising a processor (232), a memory unit (233), a stimulation unit (235) configured to stimulate a human or animal heart (1), a detection unit (231) configured to detect an electric signal of the same heart (1), a proximal electrode pole (213), a first distal electrode pole (203), and a second distal electrode pole (223), wherein the proximal electrode pole (213), the first distal electrode pole (203), and the second distal electrode pole (223) form part of the stimulation unit (235) and the detection unit (231), **characterized**
**in that** the proximal electrode pole (213) is designed and arranged to be implanted within an atrium (2) of the heart (1) to be stimulated, the first distal electrode pole (203) is designed and arranged to be implanted within a septum (13) of the heart (1) to be stimulated, and the second distal electrode pole (223) is designed and arranged to be implanted within an apex (15) of the heart (1) to be stimulated and in that the memory unit (233) comprises a computer-readable program that causes the processor (232) to perform the following steps when being executed on the processor (232):
a) detecting (500), with the proximal electrode pole (213), an intrinsic atrial contraction of the heart (1) to be stimulated or stimulating (500), with the proximal electrode pole (213), the atrium (2) of the heart (1) to be stimulated;
b) detecting (530), with the first distal electrode pole (203) and with the second distal electrode pole (223), an intrinsic ventricular contraction of the heart (1) to be stimulated;
c) determining (530) a first intrinsic atrioventricular conduction time between i) the intrinsic atrial contraction or the stimulation of the atrium and ii) the intrinsic ventricular contraction detected with the first distal electrode pole (203); and determining (530) a second intrinsic atrioventricular conduction time between i) the intrinsic atrial contraction or the stimulation of the atrium and iii) the intrinsic ventricular contraction detected with the second distal electrode pole (223);
d) setting (550) a stimulated atrioventricular conduction time for stimulating the ventricle (3, 5) of the heart (1) to be stimulated with the first distal electrode pole (203) and/or the second distal electrode pole (223), the stimulated atrioventricular conduction time being shorter than the shorter of the first intrinsic atrioventricular conduction time and the second intrinsic atrioventricular conduction time.

2. Implantable medical device according to claim 1, **characterized in that** the proximal electrode pole is configured to be implanted within the right atrium, and **in that** the computer-readable program causes the processor (232) to perform the following steps when being executed on the processor (232):
a) detecting (500), with the proximal electrode pole (213), an intrinsic right atrial contraction of the heart (1) to be stimulated or stimulating (500), with the proximal electrode pole (213), the right atrium (2) of the heart (1) to be stimulated;
b) detecting (530), with the first distal electrode pole (203) and with the second distal electrode pole (223), an intrinsic right ventricular contraction of the heart (1) to be stimulated;
c) determining (530) the first intrinsic atrioventricular conduction time between i) the intrinsic right atrial contraction or the stimulation of the right atrium and ii) the intrinsic right ventricular contraction detected with the first distal electrode pole (203); and determining (530) the second intrinsic atrioventricular conduction time between i) the intrinsic right atrial contraction or the stimulation of the right atrium and iii) the intrinsic right ventricular contraction detected with the second distal electrode pole (223);
d) setting (550) the stimulated atrioventricular conduction time for stimulating the left ventricle (5) of the heart (1) to be stimulated with the first distal electrode pole (203) and/or the second distal electrode pole (223), the stimulated atrioventricular conduction time being shorter than the shorter of the first intrinsic atrioventricular conduction time and the second intrinsic atrioventricular conduction time.

3. Implantable medical device according to claim 1 or 2, **characterized in that** the proximal electrode pole (213) is a single electrode pole or a bipole.

4. Implantable medical device according to any of the preceding claims, **characterized in that** the first distal electrode pole (203) or the second distal electrode pole (223) is a single electrode pole or a bipole.

5. Implantable medical device according to any of the preceding claims, **characterized in that** the computer-readable program causes the processor (232) to subtract a predeterminable absolute value from the shorter of the first intrinsic atrioventricular conduction time and the second intrinsic atrioventricular conduction time for defining the stimulated atrioventricular conduction time.

6. Implantable medical device according to any of the preceding claims, **characterized in that** the computer-readable program causes the processor (232) to subtract a predeterminable relative value from the shorter of the first intrinsic atrioventricular conduction time and the second intrinsic atrioventricular conduction time for defining the stimulated atrioventricular conduction time.

7. Implantable medical device according to any of the preceding claims, **characterized in that** the computer-readable program causes the processor (232) to regularly repeat steps a) to d) after a predeterminable number of cardiac cycles and/or after a predeterminable time interval.

8. Implantable medical device according to any of the preceding claims, **characterized in that** the computer-readable program causes the processor (232) to increase the stimulated atrioventricular conduction time to an amount that is longer than an expected intrinsic atrioventricular conduction time when step c) is to be performed.

9. Implantable medical device according to any of the preceding claims, **characterized in that** the computer-readable program causes the processor (232) to detect the intrinsic ventricular contraction by evaluating a far-field electrocardiogram that is measured between i) the first distal electrode pole (203) or the second distal electrode pole (223) and ii) a housing (23) of the implantable medical device (22).

10. Implantable medical device according to any of the preceding claims, **characterized in that** the implantable medical device comprises a shock coil (210) located proximally of the first distal electrode pole (203) or proximally of the second distal electrode pole (223) and **in that** the computer-readable program causes the processor (232) to detect the intrinsic ventricular contraction by evaluating a far-field electrocardiogram that is measured between the shock coil (210) and a housing (23) of the implantable medical device (22).

11. Implantable medical device according to claim 9 or 10, **characterized in that** the computer-readable program causes the processor (232) to use an earliest time point of a ventricular excitation as measure for the intrinsic ventricular contraction.

12. Implantable medical device according to the preceding claim, **characterized in that** the computer-readable program causes the processor (232) to determine the earliest time point of a ventricular excitation by a morphologic signal evaluation of the far-field electrocardiogram.

13. Implantable medical device according to any of the preceding claims, **characterized in that** the computer-readable program causes the processor (232) to perform step d) only if at least one of the determined first intrinsic atrioventricular conduction time and the determined second intrinsic atrioventricular conduction time lies within a predeterminable range and to set the stimulated atrioventricular conduction time to a predeterminable fixed value if both the determined first intrinsic atrioventricular conduction time and the determined second intrinsic atrioventricular conduction time lie outside the predeterminable range.

14. Implantable medical device according to any of the preceding claims, **characterized in that** the computer-readable program causes the processor (232) to set the stimulated atrioventricular conduction time to a value lying within a predeterminable range.

15. Method for operating an implantable medical device (22) according to any of the preceding claims, the method comprising the following steps:
a) detecting (500), with the proximal electrode pole (213), an intrinsic atrial contraction of the heart (1) to be stimulated or stimulating (500), with the proximal electrode pole (213), the atrium (2) of the heart (1) to be stimulated;
b) detecting (530), with the first distal electrode pole (203) and with the second distal electrode pole (223), an intrinsic ventricular contraction of the heart (1) to be stimulated;
c) determining (530) a first intrinsic atrioventricular conduction time between the intrinsic atrial contraction or the stimulation of the atrium and the intrinsic ventricular contraction detected with the first distal electrode pole (203); and determining (530) a second intrinsic atrioventricular conduction time between the intrinsic atrial contraction or the stimulation of the atrium and the intrinsic ventricular contraction detected with the second distal electrode pole (223);
d) setting (550) a stimulated atrioventricular conduction time for stimulating a ventricle (3, 5) of the heart (1) to be stimulated with the first distal electrode pole (203) and/or the second distal electrode pole (223), the stimulated atrioventricular conduction time being shorter than the shorter of the first intrinsic atrioventricular conduction time and the second intrinsic atrioventricular conduction time.
